# EUROPÄISCHE PATENTSCHRIFT

(11) **EP 2 510 955 B1**
(45) Veröffentlichungstag und Bekanntmachung des Hinweises auf die Patenterteilung: **11.01.2017**
(21) Anmeldenummer: 11003077.2
(22) Anmeldetag: 12.04.2011
(51) Int. Cl.: A61L 24/00, A61L 24/06

(54) **Stoffgemisch und Verwendung eines Stoffgemisches zur Herstellung eines Knochenklebers**
Composition and use of a composition to manufacture a bone adhesive
Composition et utilisation d'un composition pour la fabrication d'un ciment osseux

(43) Veröffentlichungstag der Anmeldung: 17.10.2012
(73) Patentinhaber: Karl Leibinger Medizintechnik GmbH & Co. KG, 78570 Mühlheim/Donau (DE)
(72) Erfinder: Leibinger, Christian, 78462 Konstanz (DE); Maurer, Peter, 27476 Cuxhaven (DE); Schubert, Johannes, 06193 Petersberg/OT Teicha (DE); Wolfram, Tobias, 63303 Dreieich (DE)
(74) Vertreter: Winter, Brandl, Fürniss, Hübner, Röss, Kaiser, Polte - Partnerschaft mbB

(56) Entgegenhaltungen:
- EP-A1- 1 639 987
- EP-A2- 1 066 813
- WO-A1-02/40398
- WO-A1-03/090647
- WO-A2-03/057792
- FR-A1- 2 344 281
- Fabio Mitugui Nihi ET AL: "In Vitro Assessment of Solvent Evaporation from Commercial Adhesive Systems Compared to Experimental Systems", Braz Dent J, vol. 20, no. 5, 31 December 2009 (2009-12-31), pages 396-402, XP055284986,

## Beschreibung

Die Erfindung betrifft ein Stoffgemisch zum Verbinden von einem ersten Körperteil eines menschlichen oder tierischen Lebewesens, mit einem zweiten Körperteil eines solchen Lebewesens, beinhaltend eine Mischung aus Bestandteilen der Gruppe aus Phosphonsäureacrylat, Hydroxyethylmethacrylat, Dimethylacrylat, Siliciumdioxid, einem Alkohol sowie Katalysatoren, Stabilisatoren, Initiatoren und Enbucrilat, wobei der erste Körperteil und der zweite Körperteil ein Knochenabschnitt ist, wobei die Mischung wenigstens Phosphonsäureacrylat, Hydroxyethylmethacrylat, Dimethacrylat, Siliciumdioxid, einen Alkohol, einen Katalysator und/oder einen Initiator und einen Stabilisator umfasst und weniger als 11 % der Gesamtmischung Phosphonsäureacrylat, weniger als 15 % Hydroxyethylmethacrylat, weniger als 23 % Dimethacrylat, und weniger als 20 % Alkohol in der Mischung enthalten ist.

Aus dem Stand der Technik sind Gewebekleber, wie Histoacryl bekannt. Diese Gewebekleber werden zum nahtlosen Verschluss kleiner und nicht unter Spannung stehender Hautwunden eingesetzt. Sie werden als sicherer und schneller Wundverschluss verwendet. Das unter dem Markennamen Histoacryl bekannte Medikament enthält auch einen von der amerikanischen Gesundheitsbehörde FDA zugelassenen Farbstoff, der es ermöglicht, die aufgetragene Schichtdicke zu beurteilen. Mit diesem Medikament werden glatte und frische Hautwunden verklebt. Es soll jedoch nicht an inneren Organen, der Hirnoberfläche, dem zentralen Nervensystem und an Blutgefäßen, also im Inneren des Körpers angewendet werden.

Es ist bekannt, dass Histoacryl mit dem Erreichen guter kosmetischer Ergebnisse, einer hohen mechanischen Festigkeit, der Möglichkeit der Verwendung ohne Lokalanästhesie und einem Einsatz ohne Ziehen von Fäden beworben wird. Als Vorteile werden ebenfalls das Entfallen zusätzlicher Traumata, etwa durch Stichkanäle, das Vorhandensein kurzer Behandlungszeiten und das Bilden eines Schutzfilms gegen ein Eindringen von Bakterien in den Vordergrund gestellt.

Aus dem Stand der Technik ist auch bekannt, dass ein unter dem Markennamen Excite von der Firma Ivoclar Vivadent in Ellwangen, Deutschland, vertriebener Kleber an Zähnen, also im Dentin eingesetzt wird. Dieser Kleber ist in unterschiedlichen Ausgestaltungen auf dem Markt erhältlich, insbesondere als Einkomponenten-Haftvermittler.

Während für den in Histoacryl eingesetzten Wirkstoff, nämlich N-Butyl-2-Zyanoacrylat bekannt ist, dass dieser hauptsächlich im äußeren Hautbereich zu verwenden ist und nicht als Weichgewebeklebstoff zu verwenden ist, ist der Anwendungsbereich von Excite auf den Zahnbereich beschränkt.

Im Bereich von Knochen sind bisher hauptsächlich mechanische Verbindungsunterstützer zum Einsatz gekommen. Es werden unter Knochen nicht nur solche natürlichen Vorkommens sondern auch synthetischer Natur verstanden.

So wurden zur Verbindung von Knochen bisher metallische Verbindungselemente, wie etwa Titanklammern verwendet. Alternativ oder zusätzlich wurden resorbierbare Pins eingesetzt, die unter Verwendung einer Strahlenanregung, wie etwa Ultraschallanregung, zum Verschmelzen mit dem Knochen angeregt wurden. Ein solch bekanntes Verfahren ist z.B. das "SonicWeldRx®"-Verfahren.

Es ist aber in der Medizin das Bedürfnis einer verbesserten Verbindung zum Knochen zu erkennen. Solche Knochen können entweder im lebenden Organismus vorhanden sein, oder aber außerhalb des lebenden Organismus vorhanden sein, insbesondere auch Bestandteil eines toten Körpers sein.

Es ist daher die Aufgabe der Erfindung, eine kostengünstigere und einfachere Verbindung von Knochen möglich zu machen, insbesondere unter zur Verfügung stellen eines entsprechenden Stoffgemisches, das hierbei einsetzbar ist.

Das Stoffgemisch bzw. der Stoff soll insbesondere im kranialen Bereich eines Körpers, bspw. im Kieferknochen - oder Schädelknochenbereich einsetzbar sein.

Diese Aufgabe wird erfindungsgemäß durch den Gegenstand des Patentanspruchs 1 gelöst, der insbesondere an einem ersten Körperteil und einem zweiten Körperteil eingesetzt ist, die als Knochenabschnitte ausgebildet sind. Solche Knochenabschnitte können punktförmig oder flächig ausgebildet sein.

Diese Aufgabe wird speziell auch durch den Gegenstand des Patentanspruchs 1 gelöst, der zur spezifischen Anwendung in einem Verfahren zur chirurgischen und/oder therapeutischen Behandlung des tierischen oder menschlichen Körpers vorgesehen ist, wobei die spezifische Anwendung das Aufbringen auf einen oder mehrere Knochen und/oder Knochenfragmente des Körpers ist, und der Kleber vorzugsweise eine räumlich-vernetzende Funktion zwischen den Knochen und/oder Knochenfragmenten darstellt.

Die Aufgabe wird auch durch die Verwendung eines Stoffgemisches gemäß des Patentanspruchs 1 zur Herstellung eines Klebers zum Einsatz in einem chirurgischen und/oder therapeutischen Verfahren gelöst, wobei in dem Verfahren der Kleber auf einen Knochen aufgebracht wird.

Phosphatgruppen zeigen eine hohe Affinität zu gewissen positiv geladenen Ionen. Es ist zwar bekannt, solche Phosphatgruppen in Dentinadhäsiven einzusetzen, doch ist eine erfinderische Neuerung gerade im Einsatz am Knochen zu sehen. Hierzu soll die Phosphorsäuregruppe an eine Metacrylat-Gruppe gekoppelt werden. Wegen ihrer Affinität zu positiv geladenen Ionen bindet die Phosphorsäuregruppe Calcium des Knochens, während die Metacrylat-Gruppe den chemischen Verbund mit den weiteren polymerisierbaren Komponenten des Adhäsivs erlaubt.

Die eine Phosphonsäureverbindung umfassende Stoffmischung, wirkt als Haftmonomer. Sie ist wesentlich stabiler, weil das Phosphoratom direkt an ein Kohlenstoffatom gebunden wird.

Die Phosphonsäureverbindung kann als säurehaltiges Monomer in einem Einflaschen-Adhäsiv verwendet werden.

Im Vergleich zu anderen Adhäsiven weist die neue Mischung einen sehr hohen Anteil an Monomeren auf. Während andere Adhäsive bis zu 80 % Lösungsmittel enthalten, kann der entsprechende Anteil bei nur 20 % liegen. Ein hoher Gehalt an Monomeren, bis über 79 %, führt zu einer besonders gut polymerisierten Adhäsivschicht, die auch großflächig aufgetragen werden kann.

Für den Anwender bedeutet dies dann, dass die Adhäsivschicht nicht sehr stark verblasen werden muss, um Lösungsmittel zu verdampfen, was auch die Gefahr mit sich bringt, Adhäsivmonomere wegzublasen. Dies kann ansonsten zu einer unzureichenden Schichtdicke führen. Der Wirkstoff soll nur so leicht verblasen werden, dass sich das Material zu einer homogenen Schicht verteilen kann.

Es ist von Vorteil, dass auf Aceton als Lösungsmittel verzichtet werden kann, da sich Aceton durch eine besonders hohe Flüchtigkeit auszeichnet, was zwar positive Wirkungen auf die Trocknung der aufgetragenen Adhäsivschicht hat, allerdings nur auf feuchtem Untergrund funktioniert, da sonst keine Haftung aufgebaut werden kann.

Wasserhaltige Adhäsive andererseits sind zwar unempfindlich bzgl. des Feuchtigkeitszustand der Anwendungsumgebung, allerdings muss die Adhäsivschicht ausreichend getrocknet werden, um das Wasser zu entfernen. Hier bietet ein Alkohol, wie insbesondere Ethanol einen besonderen Vorteil, nämlich die Verbindung der positiven Eigenschaften von Aceton und Wasser. Ein weiterer Vorteil von Ethanol gegenüber Aceton ist, dass er bei geöffneter Flasche wesentlich weniger verdunstet, als bei Aceton, und sich darum die Viskosität des Adhäsivs nicht spürbar verändert, bis eine Flasche aufgebraucht ist. Es ist daher von Vorteil, wenn der Alkohol Ethanol ist.

Weitere vorteilhafte Ausgestaltungsformen sind in den Unteransprüchen beansprucht und werden nachfolgend näher erläutert.

So ist es von Vorteil, wenn die Knochenabschnitte voneinander vollständig getrennt sind oder miteinander über eine Brücke gemeinsamen Materials miteinander verbunden sind. Auf diese Weise wird der Anwendungsbereich vergrößert.

Wenn die Mischung wenigstens Phosphonsäureacetylat, Hydroxyethylmethacrylat, Dimethacrylat, Siliciumdioxid, einen Alkohol wie Ethanol, einen Katalysator und/oder einen Initiator und einen Stabilisator umfasst, so ist bei einer solchen Mischung besonders wenig Nebenwirkung zu erwarten.

Ein weiteres vorteilhaftes Ausführungsbeispiel ist dadurch gekennzeichnet, dass zusätzlich Bis-GMA enthalten ist. Auch ist es von Vorteil, wenn das Siliciumdioxid als hochdisperses Siliciumdioxid ausgebildet ist.

Als besonders vorteilhaft hat es sich herausgestellt, wenn die Teilelemente Phosphonsäureacetylat, Hydroxyethylmethacrylat, Bis-GMA und Dimethacrylat, zwischen 73% und 80 % an der Gesamtmasse beitragen. Als besonders vorteilhaft haben sich Werte von 79,1%, 73,3% und 73,6% herausgestellt.

Ferner hat es sich als vorteilhaft herausgestellt, wenn das hochdisperse Siliciumdioxid 0,5 % der gesamten Mischung stellt.

Ferner hat es sich als vorteilhaft herausgestellt, wenn Ethanol einen Gesamtbeitrag von 19% bis weniger als 20% zusteuert.

Die Menge der Katalysatoren und Stabilisatoren sowie Initiatoren sollte zwischen 0,8% und 1,8% liegen, insbesondere bei 0,9% oder 1,7 % liegen.

Die Prozentangaben beziehen sich immer auf Gewichts-Prozente.

Auch ist es von Vorteil, wenn osteoinduktive Ionen und/oder Wachstumsfaktoren und/oder antibiotische Wirkstoffe in dem Stoffgemisch enthalten sind, da dann, etwa über die Verwendung von BMPs, eine schnellere Knochenheilung oder Knochenmodulation erreicht werden kann.

Es ist ferner von Vorteil, wenn in entsprechender Verwendung des Stoffgemisches, in einem ersten Schritt der Kleber auf einen ersten Knochenabschnitt und/oder einen zweiten Knochenabschnitt aufgebracht wird.

Wenn in einem vorzugsweise auf den ersten Schritt folgenden zweiten Schritt der erste Knochenabschnitt so an dem zweiten Knochenabschnitt angelegt wird, dass dazwischen der Kleber befindlich ist, so lässt sich eine besonders dauerhafte Naht erreichen.

Es ist zu erwarten, dass zumindest eine osteokonduktive oder sogar eine osteoinduktive Wirkung erreicht wird. Eine Gerüstwirkung für Osteoplasten ist ebenfalls erwartbar. Eventuell werden wahrscheinlich Wachstumsfaktoren und BMPs aktiviert.

Auf jeden Fall zeigen sich Vorteile bzgl. einer hohen Biokompatibilität, guten Resorbierbarkeit, einer schnellen und operateur-freundlichen Anwendbarkeit, einer minimalen Gewebetraumatisierung und einer hohen Belastbarkeit. Allerdings ist es insbesondere von Vorteil, die Erfindung im Bereich von wenig belasteten Knochenarealen einzusetzen.

Wenn der erste Knochenabschnitt oder der zweite Knochenabschnitt ein synthetisches Produkt ist, so lassen sich Verbesserungen bezüglich der Osteoinduktivität beziehungsweise Osteokonduktivität erzielen.

## Patentansprüche

1. Stoffgemisch zum Verbinden von einem ersten Körperteil eines menschlichen oder tierischen Lebewesens, mit einem zweiten Körperteil eines solchen Lebewesens, beinhaltend eine Mischung aus Bestandteilen der Gruppe aus Phosphonsäureacrylat, Hydroxyethylmethacrylat, Dimethylacrylat, Siliciumdioxid, einem Alkohol sowie Katalysatoren, Stabilisatoren, Initiatoren und Enbucrilat, wobei der erste Körperteil und der zweite Körperteil ein Knochenabschnitt ist, wobei die Mischung wenigstens Phosphonsäureacrylat, Hydroxyethylmethacrylat, Dimethacrylat, Siliciumdioxid, einen Alkohol, einen Katalysator und/oder einen Initiator und einen Stabilisator umfasst und weniger als 11 % der Gesamtmischung Phosphonsäureacrylat, weniger als 15 % Hydroxyethylmethacrylat, weniger als 23 % Dimethacrylat, und weniger als 20 % Alkohol in der Mischung enthalten ist.

2. Stoffgemisch nach Anspruch 1, **dadurch gekennzeichnet, dass** die Knochenabschnitte voneinander vollständig getrennt sind oder miteinander über eine Brücke gemeinsamen Materials verbunden sind.

3. Stoffgemisch nach einem der Ansprüche 1 oder 2, **dadurch gekennzeichnet, dass** der Alkohol Ethanol ist.

4. Stoffgemisch nach einem der Ansprüche 1 bis 3, **dadurch gekennzeichnet, dass** zusätzlich Bis-GMA enthalten ist.

5. Stoffgemisch nach einem der Ansprüche 1 bis 4, **dadurch gekennzeichnet, dass** das Siliciumdioxid als hochdisperses Siliciumdioxid ausgebildet ist.

6. Stoffgemisch nach einem der vorhergehenden Ansprüche, **dadurch gekennzeichnet, dass** osteoinduktive Ionen und/oder Wachstumsfaktoren und/oder antibiotische Wirkstoffe enthalten sind.

## Claims

1. Mixture of substances for connecting a first part of the body of a human or animal organism to a second part of the body of such an organism, comprising a mixture of constituents from the group of phosphonic acid acrylate, hydroxyethyl methacrylate, dimethacrylate, silicon dioxide, an alcohol and catalysts, stabilisers, initiators and enbucrilate, wherein the first part of the body and the second part of the body is a bone section, wherein the mixture comprises at least phosphonic acid acrylate, hydroxyethyl methacrylate, dimethacrylate, silicon dioxide, an alcohol, a catalyst and/or an initiator and a stabiliser and the mixture comprises less than 11 % of the total mixture of phosphonic acid acrylate, less than 15 % of hydroxyethyl methacrylate, less than 23 % of dimethacrylate and less than 20 % of alcohol.

2. Compositions according to claim 1, **characterised in that** the bone sections are separated completely from one another or connected to one another via a bridge of common material.

3. Compositions according to one of claims 1 or 2, **characterised in that** the alcohol is ethanol.

4. Compositions according to one of claims 1 to 3, **characterised in that** it additionally comprises bis-GMA.

5. Compositions according to one of claims 1 to 4, **characterised in that** the silicon dioxide is formed as highly disperse silicon dioxide.

6. Compositions according to one of the preceding claims, **characterised in that** it comprises osteoinductive ions and/or growth factors and/or antibiotic active substances.

## Revendications

1. Mélange de substances pour la liaison d'une première partie corporelle d'un être vivant humain ou animal, à une deuxième partie corporelle de cet être vivant, comportant un mélange de parties de composant du groupe comprenant un acrylate d'acide phosphonique, un méthacrylate d'hydroxyéthyle, un diméthacrylate, du dioxyde de silicium, un alcool et des catalyseurs, des stabilisants, des initiateurs et de l'enbucrilate, dans lequel la première partie corporelle et la deuxième partie corporelle sont un segment osseux, dans lequel le mélange comprend au moins un acrylate d'acide phosphonique, un méthacrylate d'hydroxyéthyle, un diméthacrylate, du dioxyde de silicium, un alcool, un catalyseur et/ou un initiateur et un stabilisant, et le mélange total contient moins de 11 % d'acrylate d'acide phosphonique, moins de 15 % de méthacrylate d'hydroxyéthyle, moins de 23 % de diméthacrylate et moins de 20 % d'alcool.

2. Mélange de substances selon la revendication 1, **caractérisé en ce que** les segments osseux sont complètement séparés l'un de l'autre ou sont reliés l'un à l'autre par un pont d'un matériau commun.

3. Mélange de substances selon l'une des revendications 1 ou 2, **caractérisé en ce que** l'alcool est de l'éthanol.

4. Mélange de substances selon l'une des revendications 1 à 3, **caractérisé en ce qu'**il contient en outre du bis-GMA.

5. Mélange de substances selon l'une des revendications 1 à 4, **caractérisé en ce que** le dioxyde de silicium est formé en tant que dioxyde de silicium hautement dispersé.

6. Mélange de substances selon l'une des revendications précédentes, **caractérisé en ce qu'**il contient des ions ostéo-inducteurs et/ou des facteurs de croissance et/ou des substances antibiotiques.
